# EUROPÄISCHE PATENTSCHRIFT

(11) **EP 2 129 410 B1**
(45) Veröffentlichungstag und Bekanntmachung des Hinweises auf die Patenterteilung: **07.09.2011**
(21) Anmeldenummer: 08717837.2
(22) Anmeldetag: 14.03.2008
(51) Int. Cl.: A61M 1/10, A61M 1/12

(54) **BLUTPUMPE MIT MIKROMOTOR**
BLOOD PUMP COMPRISING A MICROMOTOR
POMPE À SANG DOTÉE D'UN MICROMOTEUR

(30) Priorität: 24.03.2007 DE 102007014224
(43) Veröffentlichungstag der Anmeldung: 09.12.2009
(73) Patentinhaber: Abiomed Europe GmbH, 52074 Aachen (DE)
(72) Erfinder: SIESS, Thorsten, 52146 Würselen (DE); KIRCHHOFF, Frank, 52531 Übach-Palenberg (DE)
(74) Vertreter: von Kreisler Selting Werner
(86) Internationale Anmeldenummer: PCT/EP2008/053096
(87) Internationale Veröffentlichungsnummer: WO 2008/116765

(56) Entgegenhaltungen:
- WO-A-02/41935
- DE-A1- 10 005 432
- US-A- 4 957 504
- US-A- 5 302 874

## Beschreibung

Die Erfindung betrifft eine Blutpumpe mit Mikromotor für den intrakorporalen Einsatz und insbesondere eine über eine Gefäßschleuse intravaskulär einführbare Blutpumpe.

Im Bereich der Medizintechnik werden Blutpumpen benötigt, die in den Körper eines Patienten eingeführt werden und in einem Blutgefäß (Ader) oder im Herzen platziert werden, um die Herzfunktion zu unterstützen. Eine derartige Blutpumpe, die intravaskulär eingeführt wird, hat einen Außendurchmesser von 4-8 mm und eine rigide Länge von weniger als 35 mm. Für die kleinformatige Blutpumpe, die die erforderliche physiologische Förderleistung erbringt, ist eine hohe Pumpendrehzahl in der Größenordnung von 30.000-60.000 U/min erforderlich.

In WO 02/41935 A1 ist eine Blutpumpe mit Mikromotor beschrieben, die die oben genannten Anforderungen erfüllt. Der Mikromotor hat ein langgestrecktes rohrförmiges Gehäuse, das zugleich den Stator bildet. Das Gehäuse besteht aus einer rohrförmigen Hülse, die eine Erregerwicklung enthält, und einem die Hülse eng umgebenden Rückschlussmantel aus weichmagnetischem Material. Der Rotor enthält an einer Welle Magnete. Der Rückschlussmantel dient dazu, die von den Magneten erzeugten Magnetfelder zu konzentrieren und verlustarme Rückschlusswege für den Magnetfluss zu liefern. Dadurch werden die Streuverluste gering gehalten.

Bei dem bekannten Mikromotor besteht der Rückschlussmantel aus einem einstückigen Körper aus parallelen Ringen, die durch Schlitze getrennt sind. Benachbarte Ringe sind durch mindestens eine Brücke miteinander verbunden. Die Aufteilung des Rückschlussmantels dient dazu, die Ausbildung von Wirbelströmen zu begrenzen. Da der Rückschlussmantel nur eine sehr geringe Wandstärke von 0,2-0,4 mm hat, ist der für den Magnetfluss zur Verfügung stehende Querschnitt sehr eng. Infolgedessen wird der Rückschluss in magnetischer Sättigung betrieben. Durch die Unterbrechung des Rückschlussmantels durch zahlreiche Schlitze wird die Wirkung des Rückschlussmantels auf das Magnetfeld verringert, wodurch die magnetischen Verluste infolge der austretenden Streufelder vergrößert werden. Dies führt zu einer Erhöhung des benötigten Motorstromes und gegebenenfalls zu einer Vergrößerung des Motorvolumens.

Der Erfindung liegt die Aufgabe zugrunde, eine Blutpumpe mit Mikromotor für den intrakorporalen Einsatz mit vergrößerter Leistungsdichte zu schaffen, um die Motorabmessungen möglichst klein halten zu können.

Die erfindungsgemäße Blutpumpe mit Mikromotor ist durch den Patentanspruch 1 definiert. Sie weist einen Rückschlussmantel aus einer einstückigen ungeschlitzten Hülse aus einem Material auf, das eine ferritische Eisenlegierung mit bis zu 30% Chrom ist.

Die Erfindung basiert auf dem Gedanken, dass Wirbelströme im Rückschlussmantel in Kauf genommen werden können, wenn die elektrische Leitfähigkeit des Materials reduziert wird und somit auch die Stärke der Wirbelströme reduziert werden kann. Dies geschieht hier durch einen relativ hohen Chromanteil. Chrom bewirkt aber auch eine Erhöhung der Korrosionsbeständigkeit. Bei einer intrakorporal einzusetzenden Blutpumpe ist dies wichtig, weil Blut hochkorrosiv ist und aus Platzgründen der Rückschluss unmittelbar mit Blut in Kontakt steht. Durch die Verwendung einer ungeschlitzten Hülse werden die magnetischen Verluste des magnetischen Rückschlusses infolge optimaler Ausnutzung der vorhandenen Wandstärke (100% Rückschluss) minimiert. Dadurch verringert sich der für die erforderliche Antriebsleistung benötigte Motorstrom.

Die Wand des Rückschlussmantels ist vorzugsweise durchgehend bzw. kontinuierlich und von konstanter Wandstärke. Lediglich an den Enden der Hülse können Durchbrüche vorhanden sein, die die Verankerung an der Kunststoffwand des Stators erleichtern, jedoch praktisch keine Auswirkungen auf das Magnetfeld haben, da sie sich außerhalb des magnetischen Wirkbereichs befinden. Das Material des Rückschlussmantels ist eine Metalllegierung mit kristallinem Aufbau. Das Material ist weichmagnetisch, um die Hystereseverluste bzw. die Ummagnetisierungsverluste zu minimieren.

Vorzugsweise enthält das Material des Rückschlussmantels bis zu 8% Aluminium. Wird eine derartige Legierung, die Aluminium enthält, bei etwa 1100°C in sauerstoffhaltiger Atmosphäre getempert, so diffundiert das Aluminium an die Korngrenze der Eisenkristalle. Es oxidiert unter Sauerstoffeinfluss zu Al₂O₃. Da Aluminiumoxid ein schlechter elektrischer Leiter ist, werden die Eisenkristalle gegeneinander isoliert. Das oxidierte Aluminium bewirkt somit eine Reduzierung der Wirbelströme, so dass eine Schlitzung oder eine axiale Schichtung des Materials des Rückschlussmantels nicht mehr erforderlich ist. Dieser Effekt führt auch zu einer Al₂O₃ Schicht an der Oberfläche, so dass eine isolierende und chemisch inerte Schicht als Schutz gegen Blut entsteht.

Vorzugsweise enthält das Material des Rückschlussmantels bis zu 2% fein dispers verteiltes Yttriumoxid. Dieses trägt zudem zur Erhöhung des spezifischen Widerstands bei.

Das Material sollte kein oder möglichst wenig Kupfer enthalten, um die elektrische Leitfähigkeit möglichst klein zu halten.

Eine bevorzugte Legierung enthält
Eisen als Hauptbestandteil
Chrom bis 30%, bevorzugt 17% bis 22%
Aluminium bis 8%, bevorzugt 3% bis 6%
Yttriumoxid bis 2%
Andere bis 5%.

Die Prozentangaben sind stets Gewichtsprozente.

Im Folgenden wird unter Bezugnahme auf die Zeichnungen ein Ausführungsbeispiel der Erfindung näher erläutert.

Es zeigen:

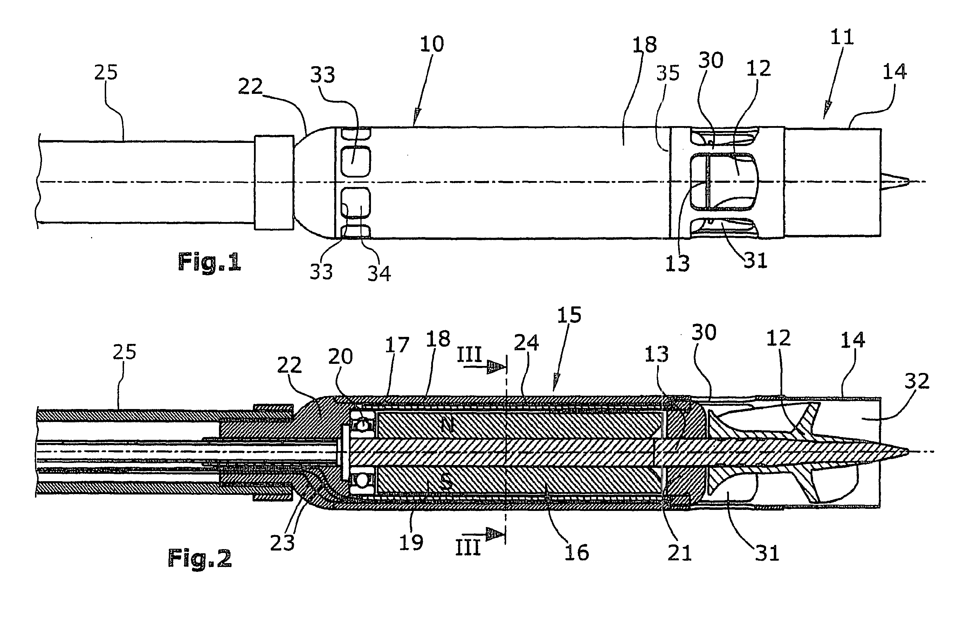

- Fig. 1: eine Seitenansicht einer Pumpe mit Mikromotor,
- Fig. 2: einen Längsschnitt durch Fig. 1, und

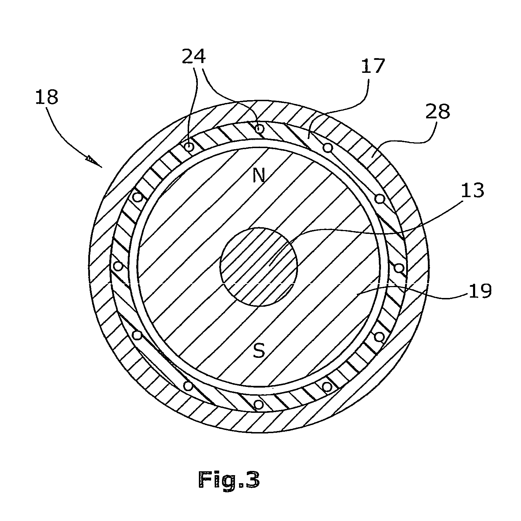

- Fig. 3: einen Querschnitt entlang der Linie III-III von Fig. 2.

Die in den Figuren dargestellte Blutpumpe entspricht weitgehend derjenigen von WO 02/41935 A1. Sie weist einen Mikromotor 10 auf, an den sich ein Pumpenteil, 11 axial anschließt. Der Pumpenteil 11 weist ein Flügelrad 12 auf, welches auf einer Welle 13 befestigt ist und in einem rohrförmigen Pumpengehäuse 14 rotiert.

Der Mikromotor 10 enthält einen Stator 15 und einen mit der Welle 13 verbundenen Rotor 16. Der Stator 15 besteht aus einer rohrförmigen Hülse 17 und einem die Hülse 17 eng umgebenden Rückschlussmantel 18. Der Rotor 16 enthält einen Magneten 19, dessen Nordpol N und Südpol S an diametral entgegengesetzten Stellen des Umfangs angeordnet sind. Der Magnet 19 ist auf der Welle 13 befestigt. Die Welle 13 ist am rückwärtigen Ende mit einem Kugellager 20 in der Hülse 17 gelagert und sie ist an dem dem Flügelrad 12 zugewandten vorderen Ende in einem Dichtlager 21 gelagert.

An das rückwärtige Ende des Stators 15 schließt sich ein Übergangsstück 22 an, das mit einem Katheter 25 verbunden werden kann. Durch das Übergangsstück 22 verlaufen Drähte 23, die mit den Erregerwicklungen 24 im Innern der Hülse verbunden sind. Die Erregerwicklung 24 wird von einem extern gesteuerten Wechselstrom durchflossen, dessen Frequenz die Drehzahl des Motors bestimmt. Die Hülse 17, welche die Erregerwicklungen 24 enthält, besteht aus einer etwa 0,2 mm starken Kunststoffschicht mit eingebetteten Drähten. Die Drähte sind zweilagig entsprechend einer vorgegebenen Konfiguration gewickelt. Zwischen dem Stator 15 und dem Rotor 16 besteht ein schmaler Spalt in der Größenordnung von einem zehntel Millimeter.

Der Rückschlussmantel 18 besteht aus einer einstückigen rohrförmigen ungeschlitzten Hülse. Er bildet zugleich die Außenhaut des Mikromotors. Erforderlichenfalls kann er noch mit einer zusätzlichen Kunststoffschicht überzogen werden.

Der Rückschlussmantel 18 umgibt eng die Hülse 17, welche die Erregerwicklungen 24 enthält.

Die Wandstärke des Rückschlussmantels beträgt etwa 0,25 mm, der Außendurchmesser beträgt 4 mm und der Innendurchmesser 3,45 mm. Die Länge des Rückschlussmantels 18 beträgt etwa 12 mm.

Der Rückschlussmantel 18 setzt sich axial durch Stege 30 fort, die vom vorderen Ende des Mikromotors nach vorne abstehen und einstückig in die Wand des Pumpengehäuses 14 übergehen. Das Pumpengehäuse 14 ist also einstückig mit dem Rückschlussmantel 18 durch Schweißen verbunden und kann daher aus einem anderen schweißbaren Material hergestellt sein. Somit können die Materialien für den Rückschluss des Motors und das Pumpengehäuse jeweils optimal auf die spezifischen Anforderungen angepasst werden. Dies bedeutet, dass das Pumpengehäuse 14 denselben Außendurchmesser und einen abweichenden Innendurchmesser vom Rückschlussmantel 18 aufweisen kann. Die Stege 30 verlaufen bei dem vorliegenden Ausführungsbeispiel parallel zur Achse des Mikromotors, so dass bereits 3 Stege eine gegen Biegung steife Verbindung ergeben.

Die zwischen den Stegen 30 gebildeten Öffnungen 31 bilden die Auslassöffnungen der Pumpe und die stirnseitige Öffnung 32 des Pumpengehäuses 14 bildet die Einlassöffnung der Pumpe. Die Pumpe kann auch in Gegenrichtung angetrieben werden, so dass die Öffnungen 31 den Einlass und die Öffnung 32 den Auslass bilden.

Bei einem bevorzugten Ausführungsbeispiel besteht der Rückschlussmantel 18 aus einem Material, das unter der Marke INCOLOY MA956 von der Firma Special Metals Corporation vertrieben wird. Dieses Material enthält die nachstehend wiedergegebene Zusammensetzung:

| Eisen | Balance |
|---|---|
| Chrom | 18.5%-21.5% |
| Aluminium | 3.75%-5.75% |
| Titan | 0.2%-0.6% |
| Kohlenstoff | 0.1% max. |
| Yttriumoxid | 0.3%-0.7% |
| Kupfer | 0.15% max. |
| Mangan | 0.30% max. |
| Cobalt | 0.3% max. |
| Nickel | 0.50% max. |
| Phosphor | 0.02% max. |

Aus diesem Material wird zunächst eine zylindrische Hülse hergestellt, die die Form und Größe des späteren Rückschlussmantels hat, und diese wird bevorzugt mehrere Stunden bei etwa 1100°C unter Sauerstoffeinwirkung getempert. Dabei oxidiert das Aluminium an den Korngrenzen teilweise zu Aluminiumoxid und es verteilt sich zwischen den Korngrenzen und entlang der Oberflächen der Hülse. Dadurch werden die Hülsenoberflächen mit einer dünnen isolierenden Keramikschicht versehen.

Die von dem Rückschlussmantel 18 gebildete Hülse enthält an ihrem proximalen Ende Fenster 33. Am distalen Ende befindet sich eine Schweißnaht 35, die den Rückschlussmantel mit dem Pumpengehäuse 14 verbindet.

Das Übergangsstück 22, das aus Kunststoff gefertigt ist, weist angeformte Noppen 34 auf, die in die Fenster 33 reichen und diese formschlüssig ausfüllen. Hierdurch wird eine stabile Verbindung zu dem rückwärtigen Katheter erreicht. Die Fenster 33 und die dazwischen befindlichen Stege sind derart zum Kugellager 20 angeordnet, dass eine Weiterleitung der Magnetfeldlinien in das Kugellager vermieden wird. Entsprechend können auch Wirbelströme im Lager vermieden werden, die gegebenenfalls zu einer Lebensdauerreduktion führen können.

Durch die Erfindung wird gegenüber einem geschlitzten Rückschlussmantel eine Verbesserung der magnetischen Eigenschaften des Rückschlussmantels erreicht, indem mehr Eisenmaterial in den Rückschluss eingebracht wird. Dadurch wird der Wirkungsgrad des Motors erhöht. Bei einer bestimmten geforderten hydraulischen Leistung der Blutpumpe kann das Motorvolumen oder der erforderliche Motorstrom reduziert werden.

Ein weiterer Vorteil der Metalllegierung besteht darin, dass das Material des Rückschlussmantels schweißbar ist. Dies hat Vorteile bei dem Zusammenbau des Motors.

Der Rückschlussmantel hat die folgenden Eigenschaften:
- ferritisch
- hohe magnetische Flussdichte
- schlechte elektrische Leitfähigkeit
- Korrosionsbeständigkeit
- Schweißbarkeit.

## Patentansprüche

1. Blutpumpe mit Mikromotor (10) für den intrakorporalen Einsatz, mit einem Stator (15), der eine eine Erregerwicklung (24) enthaltende Hülse (17) und einen die Hülse (17) umgebenden Rückschlussmantel (18) aus magnetisch leitendem Material aufweist,
**dadurch gekennzeichnet,**
**dass** der Rückschlussmantel (18) aus einer einstückigen ungeschlitzten Hülse aus einem ferritischen Material besteht, das als Hauptbestandteil Eisen und bis zu 30% Chrom enthält.

2. Blutpumpe nach Anspruch 1, **dadurch gekennzeichnet, dass** das Material des Rückschlussmantels (18) bis zu 8% Aluminium enthält.

3. Blutpumpe (10) nach Anspruch 1 oder 2, **dadurch gekennzeichnet, dass** das Material des Rückschlussmantels (18) bis zu 2% Yttriumoxid enthält.

4. Blutpumpe (10) nach einem der Ansprüche 1 bis 3, **dadurch gekennzeichnet, dass** das Material des Rückschlussmantels (18) maximal 0,2% Kupfer enthält.

5. Blutpumpe (10) nach einem der Ansprüche 1 bis 4, **dadurch gekennzeichnet, dass** das Material des Rückschlussmantels (18) außer Eisen die folgenden Bestandteile enthält:
| | |
|---|---|
| Chrom | 18,50%-21,50% |
| Aluminium | 3,75%-5,75% |
| Titan | 0,20%-0,60% |
| Kohlenstoff | max. 0,10% |
| Yttriumoxid | 0,30%-0,70% |
| Kupfer | max. 0,15% |
| Mangan | max. 0,30% |
| Cobalt | max. 0,30% |
| Nickel | max. 0,50% |
| Phosphor | max. 0,02% |

6. Blutpumpe nach einem der Ansprüche 1 bis 5, **dadurch gekennzeichnet, dass** der Außendurchmesser des Rückschlussmantels (18) nicht mehr als 4,6 mm beträgt und der Innendurchmesser mindestens 3,3 mm beträgt.

7. Blutpumpe nach einem der Ansprüche 1 bis 6, **dadurch gekennzeichnet, dass** an dem proximalen Ende des Rückschlussmantels (18) Fenster (33) vorgesehen sind.

8. Blutpumpe nach einem der Ansprüche 1 bis 7, **dadurch gekennzeichnet, dass** der Rückschlussmantel (18) mit dem Pumpengehäuse (14) durch eine Schweißnaht (35) verbunden ist.

## Claims

1. A blood pump with a micromotor (10), provided for intracorporeal use, said blood pump comprising a stator (15) accommodating a shell (17) containing an exciter coil (24), and accommodating a magnetic-reflux jacket (18) made of magnetically conductive material and surrounding said shell (17),
**characterized in**
**that** said magnetic-reflux jacket (18) consists of a one-piece unslotted shell made of a ferritic material containing iron as the main component and up to 30% chromium.

2. The blood pump according to claim 1, **characterized in that** the material of the magnetic-reflux jacket (18) contains up to 8% aluminum.

3. The blood pump (10) according to claim 1 or 2, **characterized in that** the material of the magnetic-reflux jacket (18) contains up to 2% yttrium oxide.

4. The blood pump (10) according to any one of claims 1 to 3, **characterized in that** the material of the magnetic-reflux jacket (18) contains maximally 0.2% copper.

5. The blood pump (10) according to any one of claims 1 to 4, **characterized in that** the material of the magnetic-reflux jacket (18) contains, apart from iron, the following components:
| | |
|---|---|
| chromium | 18.50% - 21.50% |
| aluminum | 3.75% - 5.75% |
| titanium | 0.20% - 0.60% |
| carbon | 0.10% max. |
| yttrium oxide | 0.30% - 0.70% |
| copper | 0.15% max. |
| manganese | 0.30% max. |
| cobalt | 0.30% max. |
| nickel | 0.50% max. |
| phosphorus | 0.02% max. |

6. The blood pump according to any one of claims 1 to 5, **characterized in that** the outer diameter of the magnetic-reflux jacket (18) does not exceed 4.6 mm and the inner diameter is at least 3.3 mm.

7. The blood pump according to any one of claims 1 to 6, **characterized in that** windows (33) are provided on the proximal end of the magnetic-reflux jacket (18).

8. The blood pump according to any one of claims 1 to 7, **characterized in that** the magnetic-reflux jacket (18) is connected to the pump housing (14) by a welding seam (35).

## Revendications

1. Pompe à sang dotée d'un micromoteur (10) destinée à l'utilisation intracorporelle, comprenant un stator (15) avec une douille (17) contenant une bobine d'excitation (24) et une enveloppe de reflux magnétique (18) en matériau conducteur de magnétisme, ladite enveloppe entourant ladite douille (17),
**caractérisée en ce que**
ladite enveloppe de reflux magnétique (18) est formée par une douille monobloc sans fentes fabriquée d'un matériau ferritique contenant du fer comme composant principal et jusqu'à 30% de chrome.

2. Pompe à sang selon la revendication 1, **caractérisée en ce que** ledit matériau de ladite enveloppe de reflux magnétique (18) contient jusqu'à 8% d'aluminium.

3. Pompe à sang (10) selon les revendications 1 ou 2, **caractérisée en ce que** ledit matériau de ladite enveloppe de reflux magnétique (18) contient jusqu'à 2% d'oxyde d'yttrium.

4. Pompe à sang (10) selon l'une quelconque des revendications 1 à 3, **caractérisée en ce que** ledit matériau de ladite enveloppe de reflux magnétique (18) contient 0,2% de cuivre au maximum.

5. Pompe à sang (10) selon l'une quelconque des revendications 1 à 4, **caractérisée en ce que** ledit matériau de ladite enveloppe de reflux magnétique (18) contient, à part du fer, les composants suivants:
| | |
|---|---|
| chrome | 18,50 - 21.50% |
| aluminum | 3.75% - 5.75% |
| titanium | 0.20% - 0.60% |
| carbone | 0.10% max. |
| oxyde d'yttrium | 0.30% - 0.70% |
| cuivre | 0.15% max. |
| manganèse | 0.30% max. |
| cobalt | 0.30% max. |
| nickel | 0.50% max. |
| phosphore | 0.02% max. |

6. Pompe à sang selon l'une quelconque des revendications 1 à 5, **caractérisée en ce que** le diamètre extérieur de ladite enveloppe de reflux magnétique (18) ne dépasse pas 4,6 mm et le diamètre intérieur est au moins 3,3 mm.

7. Pompe à sang selon l'une quelconque des revendications 1 à 6, **caractérisée en ce que** l'extrémité proximale de ladite enveloppe de reflux magnétique (18) est prévue de fenêtres (33).

8. Pompe à sang selon l'une quelconque des revendications 1 à 7, **caractérisée en ce que** ladite enveloppe de reflux magnétique (18) est connectée au boitier (14) de la pompe par une soudure (35).
